(19) Europäisches Patentamt / European Patent Office / Office européen des brevets

(11) **EP 3 256 581 B1**

(12) **EUROPEAN PATENT SPECIFICATION**

(45) Date of publication and mention
of the grant of the patent:
**09.09.2020 Bulletin 2020/37**

(51) Int Cl.:
**C12N 1/04** (2006.01)          **C12N 1/20** (2006.01)
**C12N 11/10** (2006.01)

(21) Application number: **16748521.8**

(22) Date of filing: **11.02.2016**

(86) International application number:
**PCT/CA2016/050129**

(87) International publication number:
**WO 2016/127260 (18.08.2016 Gazette 2016/33)**

(54) **IMPROVED DRY MATRIX FOR EMBEDDING VIABLE ESCHERICHIA COLI, METHOD OF MAKING SAME AND USE THEREOF**

VERBESSERTE TROCKENMATRIX ZUR EINBETTUNG LEBENSFÄHIGER ESCHERICHIA COLI, VERFAHREN ZUR HERSTELLUNG DAVON UND VERWENDUNG DAVON

MATRICE SÈCHE AMÉLIORÉE POUR L'INCORPORATION D'ESCHERICHIA COLI VIABLE, PROCÉDÉ DE FABRICATION ET UTILISATION ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **11.02.2015 US 201562114829 P**

(43) Date of publication of application:
**20.12.2017 Bulletin 2017/51**

(73) Proprietor: **Elanco Canada Limited**
**Charlottetown, PA C1E 2A7 (CA)**

(72) Inventor: **NADEAU, Eric**
**Otterburn Park, Québec J3H 5E4 (CA)**

(74) Representative: **Cabinet Becker et Associés**
**25, rue Louis le Grand**
**75002 Paris (FR)**

(56) References cited:
**WO-A1-2013/142792      WO-A2-2007/079147**
**WO-A2-2011/094469**

- **INNEKE WYNANT ET AL: "Recombinant Escherichia coli cells immobilized in Ca-alginate beads for metabolite production", BIOCATALYSIS AND BIOTRANSFORMATION., vol. 27, no. 5-6, 1 January 2009 (2009-01-01), pages 348-359, XP55487765, GB ISSN: 1024-2422, DOI: 10.3109/10242420903286141**

**Description**

**TECHNICAL FIELD**

This application generally relates to the field of improved dry matrices for embedding viable *E. coli,* method of making same and use thereof.

**BACKGROUND**

Bacterial spores are dormant life forms which can exist in a desiccated and dehydrated state indefinitely. For humans, bacterial spores are available either as over-the-counter prophylactics for mild gastrointestinal disorders, such as diarrhea, or as health foods or nutritional supplements. In the agricultural industry, bacterial spores are also receiving increasing attention as potential alternatives to antibiotics as growth promoters (Hong et al., FEMS Microbiology Reviews, 2005,29: 813-835). *Escherichia coli (E. coli)* are, however non-spore-forming, and as such, are less resistant to desiccation and/or dehydration conditions than spore-forming bacteria. In many applications, it is nevertheless necessary to preserve and store *E. coli* bacteria in a form that affords sufficient viability and/or sufficient bacterial bioactivity for a given purpose.

In this regard, various practical preservation and storage conditions for bacteria have been previously suggested.

Freeze-drying (also named lyophilisation) is often used for preservation and storage of bacteria because of the low temperature exposure during drying (Rhodes, Exploitation of microorganisms ed. Jones, DG, 1993, p. 411-439, London: Chapman & Hall). However, it has the undesirable characteristics of significantly reducing viability as well as being time and energy-intensive. Protective agents have been proposed, but the protection afforded by a given additive during freeze-drying varies with the species of micro-organism (Font de Valdez et al., Cryobiology, 1983, 20: 560-566).

Air drying such as with desiccation has also been used for preservation and storage of bacteria. While vacuum drying is a similar process as freeze-drying, it takes place at 0° - 40° C. for 30 min to a few hours. The advantages of this process are that the product is not frozen, so the energy consumption and the related economic impact are reduced. In the product point of view, the freezing damage is avoided. However, desiccation at low or ambient temperature is slow, requires extra precautions to avoid contamination, and often yields unsatisfactory viability (Lievense et al., Adv Biochem Eng Biotechnol., 1994, 51:71-89).

Encapsulating bacteria in hydrocolloid-forming polysaccharide matrix, such as Calcium-alginate (Ca-alginate) beads, has also been used for preservation and storage of bacteria in a broad and increasing range of different applications (Islam et al., J. Microbiol. Biotechnol., 2010, 20:1367-1377). To maintain the bacteria in a metabolically and physiologically competent state and thus obtain the desired benefit, it has been suggested to add to such matrices a suitable preservative formulation. Preservative formulations typically contain active ingredients in a suitable carrier and additives that aid in the stabilization and protection of the microbial cells during storage, transport and at the target zone.

Mannitol has been described as an effective preservative formulation component for Ca-alginate encapsulated bacteria during freeze-drying as it affords high bacterial viability up to 10 weeks under room temperature and water activity ($a_w$) of less than 0.2 (Efiuvwevwere et al., Appl. Microbiol. Biotechnol., 1999, 51:100-104). A synergistic mixture of trehalose and a sugar alcohol has also been described as an effective preservative formulation component for air-dried Ca-alginate encapsulated bacteria, where trehalose is used instead of sucrose for its significantly higher glass transition temperature, *i.e.*, 110° C. vs. only 65° C., respectively (U.S. 8,097,245). A synergistic mixture of carboxylic acid salts and hydrolyzed proteins has also been described as an effective preservative formulation component for freeze-dried Ca-alginate encapsulated bacteria (U.S. 2013/0,296,165). In both cases, the synergistic mixture affords an enhanced glassy structure without the need for foaming or boiling under vacuum to facilitate effective drying.

Furthermore, coating of Ca-alginate beads containing recombinant E. coli bacteria with chitosan to neutralize external surface charge of the beads has been described (Wynant et al., Biocatalysis and Biotransformation, 2009, 27(5-6): 348-359).

The development of novel formulations is, however, a challenging task and not all formulation are effective for a given bacteria (Youg et al., Biotechnol Bioeng., 2006 Sep 5;95(1):76-83).

In light of the above, there is a need to provide improved preservation and storage conditions for *E. coli* bacteria.

**SUMMARY**

The scope of the present invention is defined by the claims and any information that does not fall within the claims is provided for information only.

The present disclosure relates broadly to a viable *Escherichia coli (E. coli)* embedded in a matrix, wherein said matrix has a water activity ($a_w$) of ≤ 0.3, and wherein said matrix comprises a first polysaccharide which is a hydrocolloid-forming polysaccharide, a second polysaccharide which is different from the first polysaccharide, and a disaccharide which includes sucrose, trehalose, or a combination thereof.

The present disclosure also relates broadly to a composition for forming a matrix, said composition comprising a first polysaccharide which is a hydrocolloid-forming polysaccharide, a second polysaccharide which is different from the first polysaccharide, and a disaccharide which includes sucrose, trehalose, or a combination thereof, and an *Escherichia coli (E. coli)*.

The present disclosure also relates broadly to a method for providing a particulate comprising viable *Escherichia coli (E. coli)*.

The present disclosure also relates broadly to a matrix comprising viable *Escherichia coli (E. coli)*, wherein said matrix has a water activity ($a_w$) of $\leq 0.3$, and wherein said matrix comprises a first polysaccharide which is a hydrocolloid-forming polysaccharide, a second polysaccharide which is different from the first polysaccharide, and a disaccharide which includes sucrose, trehalose, or a combination thereof.

All features of aspects which are described in this disclosure and are not mutually exclusive can be combined with one another. Elements of one aspect can be utilized in the other aspects without further mention. Other aspects and features of the present disclosure will become apparent to those ordinarily skilled in the art upon review of the following description of specific aspects in conjunction with the accompanying Figures.

## BRIEF DESCRIPTION OF THE DRAWINGS

A detailed description of specific aspects is provided herein below with reference to the accompanying drawings in which:

Figure 1 shows a non-limiting flow diagram for preparing a bacteria culture in accordance with an aspect of the present disclosure.

Figure 2 shows a non-limiting flow diagram for drying beads with embedded *E. coli* in accordance with an aspect of the present disclosure.

Figure 3 shows a non-limiting bar graph that depicts the effect of preservation solutions S1, S2, S3 and S4 on bacterial viability following air-drying in accordance with an aspect of the present disclosure.

Figure 4 shows a non-limiting bar graph that depicts the effect of preservation solutions S1, S5, S6 and S7 on bacterial viability following air-drying in accordance with an aspect of the present disclosure.

Figure 5 shows a non-limiting bar graph that depicts the effect of preservation solutions S1, S0, S8 and S9 on bacterial viability following air-drying in accordance with an aspect of the present disclosure.

Figure 6 shows a non-limiting bar graph that depicts the effect of preservation solutions S1, S10, S11 and S12 on bacterial viability following air-drying in accordance with an aspect of the present disclosure.

Figure 7 shows a non-limiting bar graph that depicts the effect of preservation solutions S1, S13, S14 and S15 on bacterial viability following air-drying in accordance with an aspect of the present disclosure.

Figure 8 shows a non-limiting bar graph that depicts the effect of preservation solutions S1, S16, S17 and S18 on bacterial viability following air-drying in accordance with an aspect of the present disclosure.

Figure 9 shows a non-limiting bar graph that depicts the effect of preservation solutions S1 and S19 on bacterial viability following air-drying in accordance with an aspect of the present disclosure.

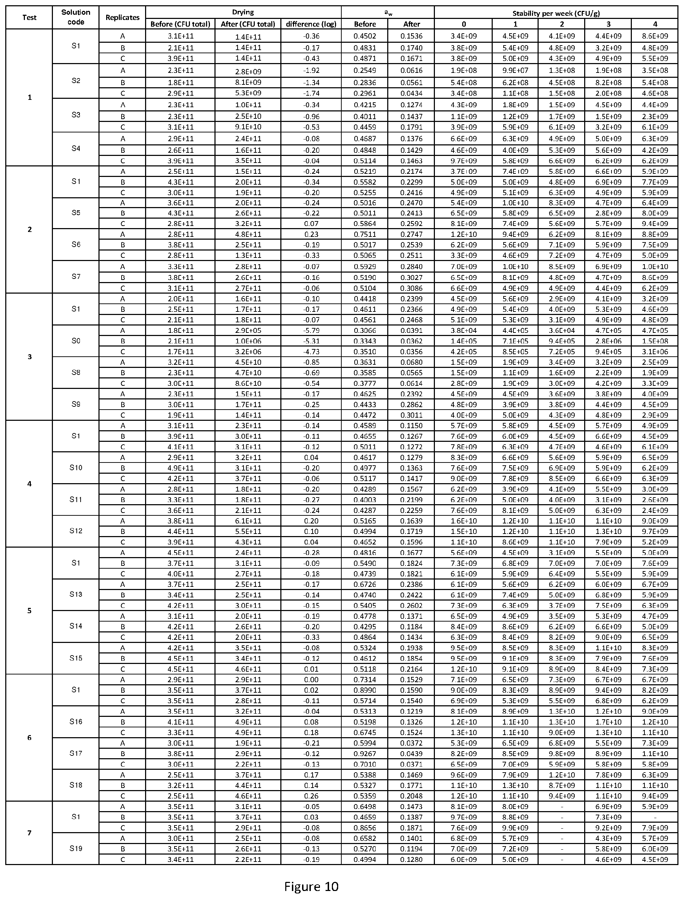

Figure 10 shows the raw data regarding Figures 3 to 9.

In the drawings, embodiments are illustrated by way of example. It is to be expressly understood that the description and drawings are only for the purpose of illustrating certain embodiments and are an aid for understanding. The scope of the claims should not be limited by the embodiments set forth in the present disclosure, but should be given the broadest interpretation consistent with the description as a whole.

## DETAILED DESCRIPTION OF EMBODIMENTS

Specific examples will now be described to illustrate the manner in which the principles of the present disclosure may be put into practice.

The herein described *E. coli* bacteria are viable bacteria, in other words, while the bacteria embedded in a dry matrix can be considered as being in a non-active state, these bacteria can be restored to an active state upon exposing the matrix to moisture.

The herein described *E. coli* bacteria comprise any recombinant or wild *E. coli* strain, or any mixtures thereof. In one embodiment, the *E. coli* is a non-pathogenic strain. In one embodiment, the non-pathogenic *E. coli* strain is the strain deposited at the International Depository Authority of Canada (IDAC) on January 21, 2005 under accession number IDAC 210105-01, or the strain deposited at the International Depositary Authority of Canada (IDAC) on June 20th 2013 and attributed accession number 200613-01, or a combination thereof.

The herein described matrix comprises a hydrocolloid-forming polysaccharide. Several polysaccharides are suitable for use as described herein, alone or in any combination thereof.

High amylose starch is a polysaccharide capable of forming firm gel after hydrating the starch granules in boiling water, dispersing the granules with the aid of high shear mixer and then cooling the solution to about 0-10° C. The firmness and strength of the gel depend on the concentration of the starch in the solution, with a maximal workable concentration of up to 10% w/v. The sliced starch gel matrix is also capable of retaining the live bacteria in the preservation mixture, and since it is mostly non-digestible by intestinal or gastric juices, the bacteria are protected from gastric destruction while within the starch matrix. The controlled release mechanism is provided by the fact that high amylose starch is readily digestible by the gut microflora at which time the delivered live bacteria are then released in their intact form.

Pectin is another suitable polysaccharide that performs very similar to high amylose starch. Pectin has an additional advantage since the strength of the pectin gel matrix can be further increased by the addition of divalent cations such as $Ca^{2+}$ that forms bridges between carboxyl groups of the sugar polymers.

Alginate is another suitable polysaccharide that can form a firm gel matrix by cross-linking with divalent cations. The alginate can be hardened into a firm gel matrix by internally cross-linking the alginate polysaccharides with a dication, e.g. $Ca^{2+}$, for example by extruding the alginate in the form of thin threads, strings, or substantially spherical beads into a $Ca^{2+}$ bath. The alginate hardens upon interaction with $Ca^{2+}$. An alternative method of preparation of the matrix is to spray atomize the mixture into a bath containing $Ca^{2+}$.

In one embodiment, the hydrocolloid-forming polysaccharide is present in the matrix in percent by weight of total dry matter at a value of from 0.1% to 20%. In one embodiment, the hydrocolloid-forming polysaccharide is present in the matrix in percent by weight of total dry matter at a value of from 0.1% to 19%, or from 0.1% to 18%, or from 0.1% to 17%, or from 0.1% to 16%, or from 0.1% to 15%, or from 0.1% to 14%, or from 0.1% to 13%, or from 0.1% to 12%, or from 1% to 12%, including any value therein.

The herein described matrix further comprises a disaccharide and a polysaccharide. The present disclosure discloses several concentrations and proportions suitable for inclusion in the matrix. In one embodiment, a suitable ratio of disaccharide / polysaccharide in wt.%/wt.% is of less than 10 or more preferably of less than 5. In one embodiment, the ratio of disaccharide / polysaccharide in wt.%/wt.% is of about 1.

In one embodiment, the disaccharide is present in the matrix in percent by weight of total dry matter at a value of from 0.1% to 90%, or from 0.1% to 75%, or from 0.1% to 50%, or from 0.1% to 35%, or from 0.1% to 20%, or from 0.1% to 15%, or from 0.1% to 10%, including any value therein.

In one non-limiting embodiment, the disaccharide includes sucrose.

In a further non-limiting embodiment, the disaccharide includes trehalose.

In one non-limiting embodiment, the polysaccharide includes maltodextrine.

In a further non-limiting embodiment, the polysaccharide includes dextran.

In a further non-limiting embodiment, the dextran has a molecular weight between 20 and 70 kDa.

In one embodiment, the matrix further includes a salt of L-glutamic acid. In one non-limiting embodiment, the salt is a sodium salt of L-glutamic acid.

The herein described matrix has a water activity ("$a_w$") which is of $0.04 \le a_w \le 0.3$, for example $0.04 \le a_w \le 2.5$, $0.04 \le a_w \le 2.0$, $0.04 \le a_w \le 1.5$, and the like. "Water activity" or "$a_w$" in the context of the present disclosure, refers to the availability of water and represents the energy status of the water in a system. Water activity may be measured according to materials and procedures known in the art, for example, using an Aqualab Water Activity Meter 4TE (Decagon Devices, Inc., U.S.A.).

There is also provided a composition for forming a matrix, the composition comprising a first hydrocolloid-forming polysaccharide, a second polysaccharide which is different from the first polysaccharide, and a disaccharide which includes sucrose, trehalose, or a combination thereof and an *Escherichia coli (E. coli)*.

There is also provided a method for providing a particulate comprising viable *Escherichia coli (E. coli)*, the method comprising providing particles comprising a first hydrocolloid-forming polysaccharide, a second polysaccharide which is different from the first polysaccharide, and a disaccharide which includes sucrose, trehalose, or a combination thereof and *E.coli* and drying said particles to water water activity $(a_w) \le 0.3$.

In one non-limiting embodiment, the viable *E. coli* sustains an $a_w$ fold reduction in the particles of at least 0.4, or at least 0.5, or at least 0.6, or at least 0.7.

**EXAMPLES**

In each of the following examples, three preservation solutions were tested along with preservation solution S1. The tests were performed in triplicates and one standard deviation was calculated according to the following formula:

$$SD = \sqrt{\frac{\sum (x - \bar{x})^2}{n}}$$

with n: number of samples and $\bar{x}$: mean of sample population.

In each of the following examples, bacterial viability was assessed by measuring the number of colony-forming units (CFU) according to protocols known in the art.

The preservation solutions used in the following examples are shown in Table 1.

Table 1

| preservation solution | Polysaccharide | Disaccharide | salt of L-glutamic acid | Ratio polysaccharide / disaccharide / salt of organic acid |
|---|---|---|---|---|
| S0 | x[1] | x | x | N/A [2] |
| S1 | dextran 40 | sucrose | yes | 5:7:1 |
| S2 | dextran 40 (5 wt%) | x | x | N/A |
| S3 | x | Sucrose (7 wt%) | x | N/A |
| S4 | dextran 40 | trehalose | yes | 5:7:1 |
| S5 | dextran 20 | sucrose | yes | 5:7:1 |
| S6 | dextran 70 | sucrose | yes | 5:7:1 |
| S7 | maltodextrine | sucrose | yes | 5:7:1 |
| S8 | dextran 40 | sucrose | yes | 10:1:1 |
| S9 | dextran 40 | sucrose | yes | 1:10:1 |
| S10 | dextran 40 (different manufacturer) | sucrose | yes | 5:7:1 |
| S11 | x | sucrose | yes | 7:1 |
| S12 | dextran 70 | trehalose | yes | 5:7:1 |
| S13 | dextran 40 | sucrose | x | 5:7 |
| S14 | dextran 40 | sucrose | yes | 5:3:1 |
| S15 | dextran 40 | sucrose | yes | 5:5:1 |
| S16 | maltodextrin | trehalose | yes | 5:7:1 |
| S17 | maltodextrin | trehalose | yes | 10:1:1 |
| S18 | maltodextrin | trehalose | yes | 1:10:1 |
| S19 | dextran 40 | maltose | yes | 5:7:1 |
| [1] x means absent [2] N/A means not applicable | | | | |

## 1. Example 1

### a. *E. coli* culture

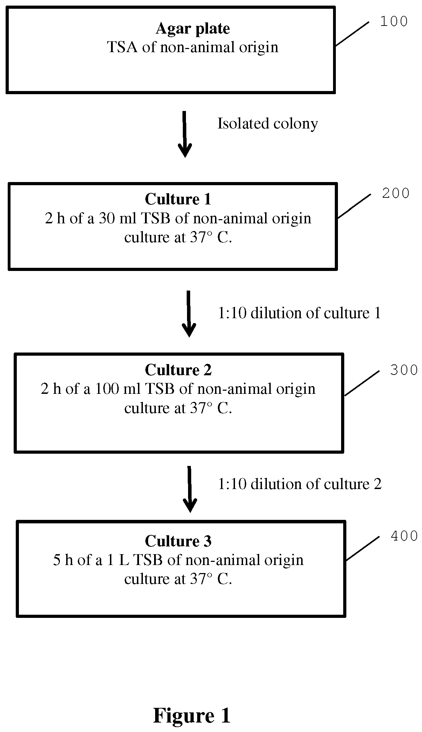

With reference to Figure 1, an *E. coli* strain was cultivated in a first step 100 on Tryptic Soy Agar of non-animal origin. Six (6) isolated colonies were then used to cultivate the *E. coli* strain in a second step 200 for 2 hours at 37° C and agitation at 200 rpm in 30 mL of Tryptic Soy Broth (TSB) of non-animal origin (for 1L of TSB: 20 g of Soy Peptone A3 SC -(Organotechnie), 2.5 g anhydrous dextrose USP -(J.T. Baker), 5 g sodium chloride USP - (J.T. Baker), and 2.5 g dibasic potassium phosphate USP -(Fisher Chemical)). The resulting Culture 1 was diluted by a factor of 10 in TSB and was then used to cultivate the *E. coli* strain in a third step 300 for 2 hours at 37° C and agitation at 200 rpm in 100 mL of TSB of non-animal origin. The resulting Culture 2 was diluted by a factor of 10 in TSB and was then used to cultivate the *E. coli* strain in a fourth step 400 for 5 hours at 37° C and agitation at 200 rpm in 1 L of TSB of non-animal origin. The resulting Culture 3 was then used to embed *E. coli* in matrix. Variations and refinements to the culture protocol herein described are possible and will become apparent to persons skilled in the art in light of the present teachings. For example, the non-pathogenic *E. coli* may also be cultivated in anaerobic conditions according to protocols known in the art (Son & Taylor, Curr. Protoc. Microbiol., 2012, 27:5A.4.1 - 5A.4.9). In preparing the beads of the subsequent examples, the non-pathogenic *E. coli* strain deposited at the International Depository Authority of Canada (IDAC) on January 21, 2005 under accession number IDAC 210105-01 may be selected.

### b. Matrix preparation

Bacto™ peptone (1.5 g, BD, Mississauga, Canada) was mixed with 1.5 L of heated water to obtain a mixture. Alginate (30 g Grindsted®, DuPont™ Danisco®, Mississauga, Canada) was slowly added to the mixture while mixing with a magnetic bar at 360 rpm. Complete solubilisation of alginate was obtained in about 3 h to obtain a 2% alginate (m/v) solution. The solution including the magnetic bar was then autoclaved under standard conditions. Variations and refinements to the matrix preparation protocol herein described are possible and will become apparent to persons skilled in the art in light of the present teachings.

### c. Embedding *E. coli* in matrix

The following was added, in order and while mixing with the magnetic bar, to the autoclaved matrix solution to obtain a slurry: 1 L of TSB of non-animal origin and, with reference to Figure 1, 0.5 L of the resulting Culture 3 of *E. coli* in 1 L of TSB of non-animal origin. The slurry was extruded into a polymerization bath (300 mM $CaCl_2$, 0.1 wt./v. % Bacto™ tryptone, 0.1 wt./v. % Bacto™ peptone, and 0.05 wt./v. % g Bacto™ yeast extract in water) to form beads using a 9 exit syringe system adapted from the Thermo Scientific™ Reacti-Vap™ Evaporators. The bath was gently stirred while injecting the slurry. The matrix beads were allowed to crosslink for about 30 minutes, and the resulting hardened beads were then harvested. Variations and refinements to the embedding protocol herein described are possible and will become apparent to persons skilled in the art in light of the present teachings.

### d. Drying and testing of embedded *E. coli*

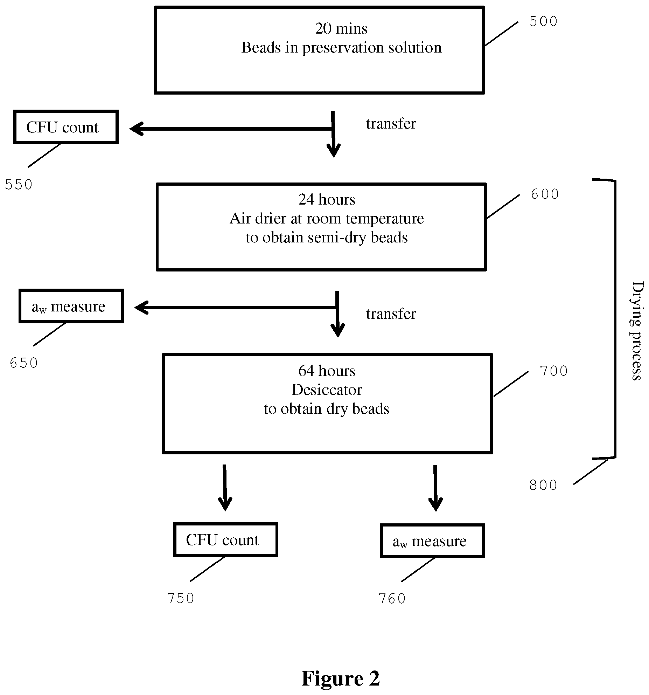

For each preservation solution the drying and testing was performed at least in triplicates. With reference to Figure 2, in a first step 500 the beads with embedded *E. coli* in the matrix were placed in a preservation solution S1, a preservation solution S2, a preservation solution S3 or a preservation solution S4 with gentle stirring for about 20 minutes. In each case, a determination of total CFU 550 was performed after soaking in the preservation solution. In a second step 600 the beads were then placed on a tray dryer in an air dryer at room temperature for about 24 h to obtain semi-dry beads. In each case, a measurement of water activity $a_w$ 650 was performed on the semi-dry beads using an Aqualab Water Activity Meter 4TE (Decagon Devices, Inc., U.S.A.). In a third step 700 the semi-dry beads were then placed in a desiccator for about 64 h, in which dry and filtered air was blown. In accordance with an aspect of the present disclosure, the drying process 800 includes at least two steps: a step 600 which includes placing beads in an air dryer for 24 hours at room temperature and to obtain semi-dry beads and a step 700 which includes placing the semi-dry beads in a desiccator for 64 hours to obtain dry beads. In each case, a determination of total CFU 750 and a measurement of water $a_w$ 760 were performed on the dry beads. Dry beads having a water activity $a_w$ of $\leq 0.3$ were obtained.

In each case, and with reference to Figure 2, the $a_w$ fold reduction was calculated according to the following:

$$a_w fold\ reduction = \frac{650 - 760}{650}$$

In each case, and with reference to Figure 2, viability loss was calculated according to the following:

$$CFU\ loss = log_{10}(550) - log_{10}(750)$$

In each case, an average viability loss and normalized average viability loss relative to the results obtained with preservation solution S1 was calculated.

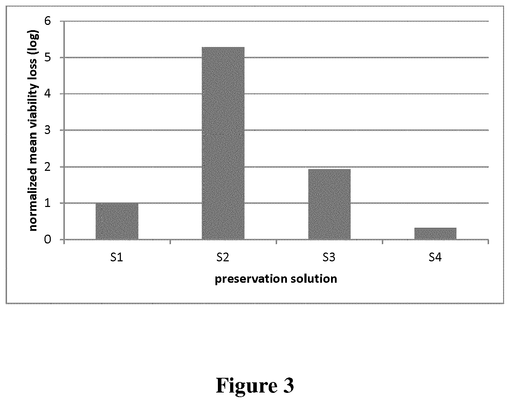

The results are shown in Figure 3. Preservation solution S4 showed a normalized average viability loss of 0.32 while sustaining a water activity of $0.142 \pm 0.004$.

A compilation of the results of Example 1 is set forth in Tables 2 and 3. These results demonstrate that the elements of preservation solution S4 provided a significant effect to the viability of the *E. coli* embedded in the dried matrix and its resistance to the drying process 700.

Table 2

| Sample | step 550 | step 750 | | |
|---|---|---|---|---|
| | average CFU | average CFU | average CFU loss ($log_{10}$) | Normalized CFU loss ($log_{10}$) |
| S1 | $3 \times 10^{11} \pm 9 \times 10^{10}$ | $1.4 \times 10^{11} \pm 4.3 \times 10^{9}$ | $0.32 \pm 0.14$ | 1 |
| S2 | $2.3 \times 10^{11} \pm 5.5 \times 10^{10}$ | $5.4 \times 10^{9} \pm 2.7 \times 10^{9}$ | $1.66 \pm 0.3$ | 5.18 |
| S3 | $2.6 \times 10^{11} \pm 4.9 \times 10^{10}$ | $7.4 \times 10^{10} \pm 4.3 \times 10^{10}$ | $0.61 \pm 0.32$ | 1.90 |
| S4 | $3.1 \times 10^{11} \pm 7 \times 10^{10}$ | $2.5 \times 10^{11} \pm 9.7 \times 10^{10}$ | $0.11 \pm 0.08$ | 0.34 |

Table 3

| Sample | step 650 | step 760 | |
|---|---|---|---|
| | average $a_w$ | average $a_w$ | $a_w$ fold reduction |
| S1 | $0.473 \pm 0.020$ | $0.165 \pm 0.010$ | 0.65 |
| S2 | $0.278 \pm 0.021$ | $0.054 \pm 0.009$ | 0.81 |
| S3 | $0.423 \pm 0.022$ | $0.150 \pm 0.026$ | 0.64 |
| S4 | $0.488 \pm 0.022$ | $0.142 \pm 0.004$ | 0.71 |

e. Incorporating dried embedded *E. coli* into a feed ("pelleting")

Protocol for incorporating dried matrix into a feed, for example in the form of a feed additive are known in the art. An illustrative example of doing such can be done, e.g., by incorporating 500 g to 1000 g of dried matrix beads into a ton of feed. If desired, the feed can also include inactivated yeast product in suitable amounts. For instance, the dried matrix beads comprising the embedded *E. coli* are mixed in a homogenization tank with all other ingredients. Preferably, the mixture is continuously mixed during the pelleting process. The mixed material is then pumped towards an extruder. Steam is then applied on the mixed material that is about to enter the extruder (i.e., hence, the temperature of the mixture increases at this stage). Suitable pressure is then applied on the mixture during its passage inside the extruder (pressure and temperature increase, point where highest temperature reached, around 75°C). The formed pellets are then expelled out of the extruder into a cooling tank (rapid temperature drops to 30-40°C followed by another cool down, to reach ambient temperature). Pelleted feed including the feed additive (matrix comprising embedded E. coli) can then be stored, for example in bags / containers. Variations and refinements to the pelleting protocol herein described are possible and will become apparent to persons skilled in the art in light of the present teachings.

2. Example 2

For each preservation solution the drying and testing was performed at least in triplicates. With reference to Figure 2, in a first step 500 beads prepared as in Example 1 were placed in a preservation solution S1, a preservation solution S5, a preservation solution S6 or a preservation solution S7 with gentle stirring for about 20 minutes. In each case, a determination of total CFU 550 was performed after soaking in the preservation solution. In a second step 600 the beads were then placed on a tray dryer in an air dryer at room temperature for about 24 h to obtain semi-dry beads. In each case, a measurement of water activity $a_w$ 650 was performed on the semi-dry beads using an Aqualab Water Activity Meter 4TE (Decagon Devices, Inc., U.S.A.). In a third step 700 the semi-dry beads were then placed in a desiccator for about 64 h, in which dry and filtered air was blown. In accordance with an aspect of the present disclosure, the drying process 800 includes at least two steps: a step 600 which includes placing beads in an air dryer for 24 hours at room temperature and to obtain semi-dry beads and a step 700 which includes placing the semi-dry beads in a desiccator for 64 hours to obtain dry beads. In each case, a determination of total CFU 750 and a measurement of water $a_w$ 760 were performed on the dry beads. Dry beads having a water activity $a_w$ of $\leq 0.3$ were obtained.

In each case, and with reference to Figure 2, the $a_w$ fold reduction was calculated according to the following:

$$a_w fold\ reduction = \frac{650 - 760}{650}$$

In each case, and with reference to Figure 2, viability loss was calculated according to the following:

$$CFU\ loss = log_{10}(550) - log_{10}(750)$$

In each case, an average viability loss and normalized average viability loss relative to the results obtained with preservation solution S1 was calculated.

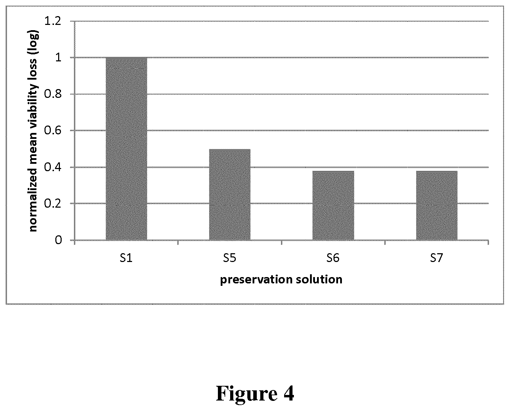

The results are shown in Figure 4. Preservation solution S7 showed a normalized average viability loss of 0.38 while sustaining a water activity of $0.298 \pm 0.013$.

A compilation of the results of Example 2 is set forth in Tables 4 and 5. These results demonstrate that the elements of preservation solution S7 provided a significant protective effect to the viability of the *E. coli* embedded in the dried matrix and its resistance to the drying process 700.

Table 4

| Sample | step 550 | step 750 | | |
|---|---|---|---|---|
| | average CFU | average CFU | average CFU loss ($log_{10}$) | Normalized CFU loss ($log_{10}$) |
| S1 | $3.3 \times 10^{11} \pm 9.2 \times 10^{10}$ | $1.8 \times 10^{11} \pm 2.7 \times 10^{10}$ | $0.26 \pm 0.07$ | 1 |
| S5 | $3.5 \times 10^{11} \pm 7.7 \times 10^{10}$ | $2.6 \times 10^{11} \pm 6 \times 10^{10}$ | $0.13 \pm 0.17$ | 0.5 |
| S6 | $3.1 \times 10^{11} \pm 5.8 \times 10^{10}$ | $2.8 \times 10^{11} \pm 1.7 \times 10^{11}$ | $0.10 \pm 0.29$ | 0.38 |
| S7 | $3.4 \times 10^{11} \pm 3.7 \times 10^{10}$ | $2.7 \times 10^{11} \pm 1 \times 10^{10}$ | $0.10 \pm 0.06$ | 0.38 |

Table 5

| Sample | step 650 | step 760 | |
|---|---|---|---|
| | average $a_w$ | average $a_w$ | $a_w$ fold reduction |
| S1 | $0.535 \pm 0.020$ | $0.230 \pm 0.012$ | 0.57 |
| S5 | $0.530 \pm 0.049$ | $0.249 \pm 0.009$ | 0.53 |
| S6 | $0.586 \pm 0.143$ | $0.260 \pm 0.013$ | 0.56 |
| S7 | $0.541 \pm 0.045$ | $0.298 \pm 0.013$ | 0.45 |

3. Example 3

For each preservation solution the drying and testing was performed at least in triplicates. With reference to Figure 2, in a first step 500 beads prepared as in Example 1 were placed in either a preservation solution S1, a preservation solution S0, a preservation solution S8 or a preservation solution S9 with gentle stirring for about 20 minutes. In each case, a determination of total CFU 550 was performed after soaking in the preservation solution. In a second step 600 the beads were then placed on a tray dryer in an air dryer at room temperature for about 24 h to obtain semi-dry beads.

In each case, a measurement of water activity $a_w$ 650 was performed on the semi-dry beads using an Aqualab Water Activity Meter 4TE (Decagon Devices, Inc., U.S.A.). In a third step 700 the semi-dry beads were then placed in a desiccator for about 64 h, in which dry and filtered air was blown. In accordance with an aspect of the present disclosure, the drying process 800 includes at least two steps: a step 600 which includes placing beads in an air dryer for 24 hours at room temperature and to obtain semi-dry beads and a step 700 which includes placing the semi-dry beads in a desiccator for 64 hours to obtain dry beads. In each case, a determination of total CFU 750 and a measurement of water $a_w$ 760 were performed on the dry beads. Dry beads having a water activity $a_w$ of $\leq 0.3$ were obtained.

In each case, and with reference to Figure 2, the $a_w$ fold reduction was calculated according to the following:

$$a_w fold\ reduction = \frac{650 - 760}{650}$$

In each case, and with reference to Figure 2, viability loss was calculated according to the following:

$$CFU\ loss = log_{10}(550) - log_{10}(750)$$

In each case, an average viability loss and normalized average viability loss relative to the results obtained with preservation solution S1 was calculated.

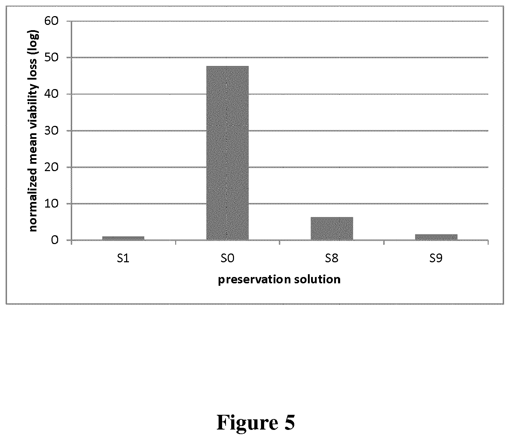

The results are shown in Figure 5.

A compilation of the results of Example 3 is set forth in Tables 6 and 7.

Table 6

| Sample | step 550 | step 750 | | |
|---|---|---|---|---|
| | average CFU | average CFU | average CFU loss ($log_{10}$) | Normalized CFU loss ($log_{10}$) |
| S1 | $2.2 \times 10^{11} \pm 2.9 \times 10^{10}$ | $1.7 \times 10^{11} \pm 9.3 \times 10^{9}$ | $0.11 \pm 0.05$ | 1 |
| S0 | $1.9 \times 10^{11} \pm 2 \times 10^{10}$ | $1.5 \times 10^{6} \pm 1.5 \times 10^{6}$ | $5.28 \pm 0.53$ | 47.7 |
| S8 | $2.8 \times 10^{11} \pm 4.6 \times 10^{10}$ | $5.9 \times 10^{10} + 2.3 \times 10^{10}$ | $0.70 \pm 0.15$ | 6.28 |
| S9 | $2.4 \times 10^{11} \pm 5.4 \times 10^{10}$ | $1.5 \times 10^{11} \pm 1.5 \times 10^{10}$ | $0.18 \pm 0.04$ | 1.64 |

Table 7

| Sample | step 650 | step 760 | |
|---|---|---|---|
| | average $a_w$ | average $a_w$ | $a_w$ fold reduction |
| S1 | $0.453 \pm 0.010$ | $0.241 \pm 0.005$ | 0.47 |
| S0 | $0.331 \pm 0.022$ | $0.037 \pm 0.002$ | 0.89 |
| S8 | $0.366 \pm 0.010$ | $0.062 \pm 0.006$ | 0.83 |
| S9 | $0.451 \pm 0.010$ | $0.275 \pm 0.032$ | 0.39 |

4. Example 4

For each preservation solution the drying and testing was performed at least in triplicates. With reference to Figure 2, in a first step 500 beads prepared as in Example 1 were placed in either a preservation solution S1, a preservation solution S10, a preservation solution S11 or a preservation solution S12 with gentle stirring for about 20 minutes. In each case, a determination of total CFU 550 was performed after soaking in the preservation solution. In a second step 600 the beads were then placed on a tray dryer in an air dryer at room temperature for about 24 h to obtain semi-dry beads. In each case, a measurement of water activity $a_w$ 650 was performed on the semi-dry beads using an Aqualab Water Activity Meter 4TE (Decagon Devices, Inc., U.S.A.). In a third step 700 the semi-dry beads were then placed in a desiccator for about 64 h, in which dry and filtered air was blown. In accordance with an aspect of the present disclosure, the drying process 800 includes at least two steps: a step 600 which includes placing beads in an air dryer for 24 hours at room temperature and to obtain semi-dry beads and a step 700 which includes placing the semi-dry beads in a desiccator for 64 hours to obtain dry beads. In each case, a determination of total CFU 750 and a measurement of water $a_w$ 760 were performed on the dry beads. Dry beads having a water activity $a_w$ of $\leq 0.3$ were obtained.

In each case, and with reference to Figure 2, the $a_w$ fold reduction was calculated according to the following:

$$a_w fold\ reduction = \frac{650 - 760}{650}$$

In each case, and with reference to Figure 2, viability loss was calculated according to the following:

$$CFU\ loss = log_{10}(550) - log_{10}(750)$$

In each case, an average viability loss and normalized average viability loss relative to the results obtained with preservation solution S1 was calculated.

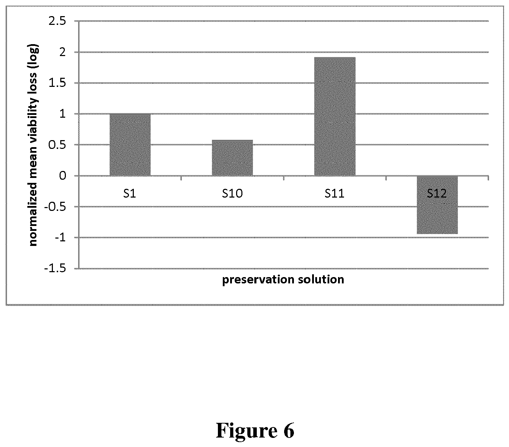

The results are shown in Figure 6. Preservation solution S7 showed a normalized average viability loss of 0.58.

A compilation of the results of Example 4 is set forth in Tables 8 and 9.

Table 8

| Sample | step 550 | step 750 | | |
|---|---|---|---|---|
| | average CFU | average CFU | average CFU loss (log$_{10}$) | Normalized CFU loss (log$_{10}$) |
| S1 | 3.7 x 10$^{11}$ $\pm$ 5.2 x 10$^{10}$ | 2.8 x 10$^{11}$ $\pm$ 4.46 x 10$^{10}$ | 0.12 $\pm$ 0.01 | 1 |
| S10 | 4 x 10$^{11}$ $\pm$ 1 x 10$^{11}$ | 3.3 x 10$^{11}$ $\pm$ 3.11 x 10$^{10}$ | 0.07 $\pm$ 0.12 | 0.58 |
| S11 | 3.3 x 10$^{11}$ $\pm$ 3.9 x 10$^{10}$ | 1.9 x 10$^{11}$ $\pm$ 1.77 x 10$^{10}$ | 0.24 $\pm$ 0.03 | 1.91 |
| S12 | 4 x 10$^{11}$ $\pm$ 2.9 x 10$^{10}$ | 5.3 x 10$^{11}$ $\pm$ 9.27 x 10$^{10}$ | -0.12 $\pm$ 0.08 | -0.94 |

Table 9

| Sample | step 650 | step 760 | |
|---|---|---|---|
| | average $a_w$ | average $a_w$ | $a_w$ fold reduction |
| S1 | 0.475 $\pm$ 0.023 | 0.123 $\pm$ 0.007 | 0.74 |
| S10 | 0.490 $\pm$ 0.026 | 0.135 $\pm$ 0.007 | 0.72 |
| S11 | 0.419 $\pm$ 0.016 | 0.201 $\pm$ 0.038 | 0.52 |
| S12 | 0.494 $\pm$ 0.026 | 0.165 $\pm$ 0.006 | 0.66 |

5. Example 5

For each preservation solution the drying and testing was performed at least in triplicates. With reference to Figure 2, in a first step 500 beads prepared as in Example 1 were placed in either a preservation solution S1, a preservation solution S13, a preservation solution S14 or a preservation solution S15 with gentle stirring for about 20 minutes. In each case, a determination of total CFU 550 was performed after soaking in the preservation solution. In a second step 600 the beads were then placed on a tray dryer in an air dryer at room temperature for about 24 h to obtain semi-dry beads. In each case, a measurement of water activity $a_w$ 650 was performed on the semi-dry beads using an Aqualab Water Activity Meter 4TE (Decagon Devices, Inc., U.S.A.). In a third step 700 the semi-dry beads were then placed in a desiccator for about 64 h, in which dry and filtered air was blown. In accordance with an aspect of the present disclosure, the drying process 800 includes at least two steps: a step 600 which includes placing beads in an air dryer for 24 hours at room temperature and to obtain semi-dry beads and a step 700 which includes placing the semi-dry beads in a desiccator for 64 hours to obtain dry beads. In each case, a determination of total CFU 750 and a measurement of water $a_w$ 760 were performed on the dry beads. Dry beads having a water activity $a_w$ of $\leq 0.3$ were obtained.

In each case, and with reference to Figure 2, the $a_w$ fold reduction was calculated according to the following:

$$a_w fold\ reduction = \frac{650 - 760}{650}$$

In each case, and with reference to Figure 2, viability loss was calculated according to the following:

$$CFU\ loss = log_{10}(550) - log_{10}(750)$$

In each case, an average viability loss and normalized average viability loss relative to the results obtained with preservation solution S1 was calculated.

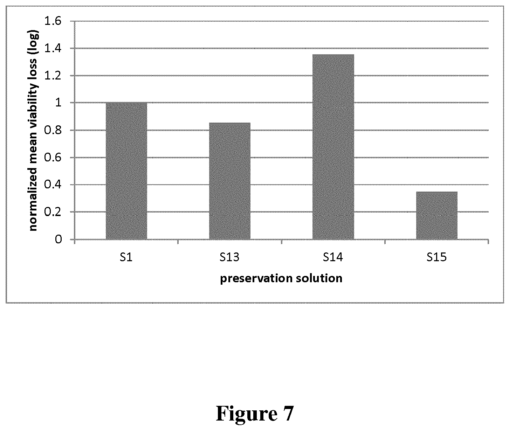

The results are shown in Figure 7. Preservation solution S7 showed a normalized average viability loss of 0.35.

A compilation of the results of Example 5 is set forth in Tables 10 and 11.

Table 10

| Sample | step 550 | step 750 | | |
|---|---|---|---|---|
| | average CFU | average CFU | average CFU loss ($log_{10}$) | Normalized CFU loss ($log_{10}$) |
| S1 | $4.1 \times 10^{11} \pm 3.8 \times 10^{10}$ | $2.7 \times 10^{11} \pm 3.44 \times 10^{10}$ | $0.18 \pm 0.10$ | 1 |
| S13 | $3.8 \times 10^{11} \pm 4.2 \times 10^{10}$ | $2.6 \times 10^{11} \pm 2.7 \times 10^{10}$ | $0.15 \pm 0.02$ | 0.85 |
| S14 | $3.8 \times 10^{11} \pm 6.1 \times 10^{10}$ | $2.2 \times 10^{11} \pm 3.55 \times 10^{10}$ | $0.24 \pm 0.08$ | 1.35 |
| S15 | $4.4 \times 10^{11} \pm 1.5 \times 10^{10}$ | $3.8 \times 10^{11} \pm 6.37 \times 10^{10}$ | $0.06 \pm 0.07$ | 0.35 |

Table 11

| Sample | step 650 | step 760 | |
|---|---|---|---|
| | average $a_w$ | average $a_w$ | $a_w$ fold reduction |
| S1 | $0.501 \pm 0.041$ | $0.177 \pm 0.008$ | 0.65 |
| S13 | $0.562 \pm 0.101$ | $0.247 \pm 0.012$ | 0.56 |
| S14 | $0.465 \pm 0.031$ | $0.133 \pm 0.013$ | 0.71 |
| S15 | $0.502 \pm 0.037$ | $0.198 \pm 0.016$ | 0.60 |

6. Example 6

For each preservation solution the drying and testing was performed at least in triplicates. With reference to Figure 2, in a first step 500 beads prepared as in Example 1 were placed in either a preservation solution S1, a preservation solution S16, a preservation solution S17 or a preservation solution S18 with gentle stirring for about 20 minutes. In each case, a determination of total CFU 550 was performed after soaking in the preservation solution. In a second step 600 the beads were then placed on a tray dryer in an air dryer at room temperature for about 24 h to obtain semi-dry beads. In each case, a measurement of water activity $a_w$ 650 was performed on the semi-dry beads using an Aqualab Water Activity Meter 4TE (Decagon Devices, Inc., U.S.A.). In a third step 700 the semi-dry beads were then placed in a desiccator for about 64 h, in which dry and filtered air was blown. In accordance with an aspect of the present disclosure, the drying process 800 includes at least two steps: a step 600 which includes placing beads in an air dryer for 24 hours at room temperature and to obtain semi-dry beads and a step 700 which includes placing the semi-dry beads in a desiccator for 64 hours to obtain dry beads. In each case, a determination of total CFU 750 and a measurement of water $a_w$ 760 were performed on the dry beads. Dry beads having a water activity $a_w$ of $\leq 0.3$ were obtained.

In each case, and with reference to Figure 2, the $a_w$ fold reduction was calculated according to the following:

$$a_w fold\ reduction = \frac{650 - 760}{650}$$

In each case, and with reference to Figure 2, viability loss was calculated according to the following:

$$CFU\ loss = log_{10}(550) - log_{10}(750)$$

In each case, an average viability loss and normalized average viability loss relative to the results obtained with preservation solution S1 was calculated.

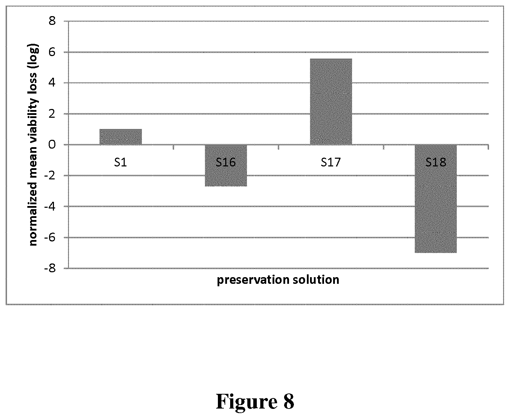

The results are shown in Figure 8.

A compilation of the results of Example 6 is set forth in Tables 12 and 13.

Table 12

| Sample | step 550 | step 750 | | |
|---|---|---|---|---|
| | average CFU | average CFU | average CFU loss ($log_{10}$) | Normalized CFU loss ($log_{10}$) |
| S1 | $3.3 \times 10^{11} \pm 3.4 \times 10^{10}$ | $3.1 \times 10^{11} \pm 4.92 \times 10^{10}$ | $0.03 \pm 0.07$ | 1 |
| S16 | $3.6 \times 10^{11} \pm 4.1 \times 10^{10}$ | $4.4 \times 10^{11} \pm 9.91 \times 10^{10}$ | $-0.07 \pm 0.11$ | -2.68 |
| S17 | $3.2 \times 10^{11} \pm 4.5 \times 10^{10}$ | $2.3 \times 10^{11} \pm 5.05 \times 10^{10}$ | $0.15 \pm 0.05$ | 5.57 |
| S18 | $2.7 \times 10^{11} \pm 3.9 \times 10^{10}$ | $4.2 \times 10^{11} \pm 4.76 \times 10^{10}$ | $-0.19 \pm 0.06$ | -6.98 |

Table 13

| Sample | step 650 | step 760 | |
|---|---|---|---|
| | average $a_w$ | average $a_w$ | $a_w$ fold reduction |
| S1 | $0.734 \pm 0.164$ | $0.155 \pm 0.003$ | 0.79 |
| S16 | $0.575 \pm 0.862$ | $0.136 \pm 0.015$ | 0.76 |
| S17 | $0.742 \pm 0.167$ | $0.039 \pm 0.004$ | 0.95 |
| S18 | $0.536 \pm 0.003$ | $0.176 \pm 0.029$ | 0.67 |

7. Example 7

For each preservation solution the drying and testing was performed at least in triplicates. With reference to Figure 2, in a first step 500 beads prepared as in Example 1 were placed in either a preservation solution S1 or a preservation solution S19 with gentle stirring for about 20 minutes. In each case, a determination of total CFU 550 was performed after soaking in the preservation solution. In a second step 600 the beads were then placed on a tray dryer in an air dryer at room temperature for about 24 h to obtain semi-dry beads. In each case, a measurement of water activity $a_w$ 650 was performed on the semi-dry beads using an Aqualab Water Activity Meter 4TE (Decagon Devices, Inc., U.S.A.). In a third step 700 the semi-dry beads were then placed in a desiccator for about 64 h, in which dry and filtered air was blown. In accordance with an aspect of the present disclosure, the drying process 800 includes at least two steps: a step 600 which includes placing beads in an air dryer for 24 hours at room temperature and to obtain semi-dry beads and a step 700 which includes placing the semi-dry beads in a desiccator for 64 hours to obtain dry beads. In each case, a determination of total CFU 750 and a measurement of water $a_w$ 760 were performed on the dry beads. Dry beads having a water activity $a_w$ of $\leq 0.3$ were obtained.

In each case, and with reference to Figure 2, the $a_w$ fold reduction was calculated according to the following:

$$a_w fold\ reduction = \frac{650 - 760}{650}$$

In each case, and with reference to Figure 2, viability loss was calculated according to the following:

$$CFU\ loss = log_{10}(550) - log_{10}(750)$$

In each case, an average viability loss and normalized average viability loss relative to the results obtained with preservation solution S1 was calculated.

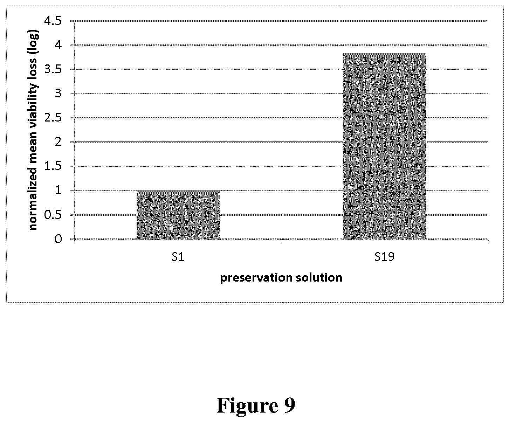

The results are shown in Figure 9.

A compilation of the results of Example 7 is set forth in Tables 14 and 15.

Table 14

| Sample | step 550 | step 750 | | |
|---|---|---|---|---|
| | average CFU | average CFU | average CFU loss ($\log_{10}$) | Normalized CFU loss ($\log_{10}$) |
| S1 | $3.5 \times 10^{11} \pm 4.4 \times 10^{8}$ | $3.2 \times 10^{11} \pm 4.13 \times 10^{10}$ | $0.03 \pm 0.05$ | 1 |
| S19 | $3.3 \times 10^{11} \pm 2.6 \times 10^{10}$ | $2.4 \times 10^{11} \pm 2.06 \times 10^{10}$ | $0.13 \pm 0.06$ | 3.83 |

Table 15

| Sample | step 650 | step 760 | |
|---|---|---|---|
| | average $a_w$ | average $a_w$ | $a_w$ fold reduction |
| S1 | $0.660 \pm 0.200$ | $0.158 \pm 0.026$ | 0.76 |
| S19 | $0.561 \pm 0.085$ | $0.129 \pm 0.010$ | 0.77 |

8. Example 8

For each preservation solution the drying and testing was performed at least in triplicates. Beads prepared as in Example 1 were placed in a preservation solution S1, a preservation solution S2, a preservation solution S3 and a preservation solution S4 with gentle stirring for about 20 minutes. The beads were then placed on a tray dryer in an air dryer at room temperature for about 24 h to obtain semi-dry beads. The semi-dry beads were then placed in a desiccator for about 64 h, in which dry and filtered air was blown. Dry beads having a water activity $a_w$ of $\leq 0.3$ were obtained.

In each case, the strain viability was tested over a period of four (4) weeks under storage conditions at 4°C by measuring the CFU/g of the dried beads. The tests were performed at least in triplicates and one standard deviation was calculated.

The results of Example 8 are shown in Table 16 where all the preservation solutions tested afforded feed additive strain stability during 4 weeks when stored at 4°C.

Table 16

| Preservation solution | Difference CFU/g after 4 weeks (log) |
|---|---|
| S1 | 0.2 |
| S2 | 0.1 |
| S3 | 0.1 |
| S4 | 0 |

9. Example 9

For each preservation solution the drying and testing was performed at least in triplicates. Beads prepared as in Example 1 were placed in a preservation solution S1, a preservation solution S5, a preservation solution S6 and a preservation solution S7 with gentle stirring for about 20 minutes. The beads were then placed on a tray dryer in an air dryer at room temperature for about 24 h to obtain semi-dry beads. The semi-dry beads were then placed in a desiccator for about 64 h, in which dry and filtered air was blown. Dry beads having a water activity $a_w$ of $\leq 0.3$ were obtained.

In each case, the strain viability was tested over a period of four (4) weeks under storage conditions at 4°C by measuring the CFU/g of the dried beads. The tests were performed at least in triplicates and one standard deviation was calculated.

The results of Example 9 are shown in Table 17 and all the preservation solutions tested afforded feed additive strain stability during 4 weeks when stored at 4°C.

Table 17

| Preservation solution | Difference CFU/g after 4 weeks (log) |
|---|---|
| S1 | 0.2 |
| S5 | 0.1 |
| S6 | 0 |
| S7 | 0.1 |

10. Example 10

For each preservation solution the drying and testing was performed at least in triplicates. Beads prepared as in Example 1 were placed in either a preservation solution S1, a preservation solution S0, a preservation solution S8 and a preservation solution S9 with gentle stirring for about 20 minutes. The beads were then placed on a tray dryer in an air dryer at room temperature for about 24 h to obtain semi-dry beads. The semi-dry beads were then placed in a desiccator for about 64 h, in which dry and filtered air was blown. Dry beads having a water activity $a_w$ of $\leq 0.3$ were obtained.

In each case, the strain viability was tested over a period of four (4) weeks under storage conditions at 4°C by measuring the CFU/g of the dried beads. The tests were performed at least in triplicates and one standard deviation was calculated.

The results of Example 10 are shown in Table 18 and all the preservation solutions tested afforded feed additive strain stability during 4 weeks when stored at 4°C.

Table 18

| Preservation solution | Difference CFU/g after 4 weeks (log) |
|---|---|
| S1 | 0 |
| S0 | 2.4 |
| S8 | 0.1 |
| S9 | 0 |

11. Example 11

For each preservation solution the drying and testing was performed at least in triplicates. Beads prepared as in Example 1 were placed in either a preservation solution S1, a preservation solution S10, a preservation solution S11 and a preservation solution S12 with gentle stirring for about 20 minutes. The beads were then placed on a tray dryer in an air dryer at room temperature for about 24 h to obtain semi-dry beads. The semi-dry beads were then placed in a desiccator for about 64 h, in which dry and filtered air was blown. Dry beads having a water activity $a_w$ of $\leq 0.3$ were obtained.

In each case, the strain viability was tested over a period of four (4) weeks under storage conditions at 4°C by measuring the CFU/g of the dried beads. The tests were performed at least in triplicates and one standard deviation was calculated.

The results of Example 11 are shown in Table 19 and all the preservation solutions tested afforded feed additive strain stability during 4 weeks when stored at 4°C.

Table 19

| Preservation solution | Difference CFU/g after 4 weeks (log) |
|---|---|
| S1 | 0.1 |
| S10 | 0.1 |
| S11 | 0.4 |
| S12 | 0.2 |

12. Example 12

For each preservation solution the drying and testing was performed at least in triplicates. Beads prepared as in Example 1 were placed in either a preservation solution S1, a preservation solution S13, a preservation solution S14 and a preservation solution S15 with gentle stirring for about 20 minutes. The beads were then placed on a tray dryer in an air dryer at room temperature for about 24 h to obtain semi-dry beads. The semi-dry beads were then placed in a desiccator for about 64 h, in which dry and filtered air was blown. Dry beads having a water activity $a_w$ of $\leq 0.3$ were obtained.

In each case, the strain viability was tested over a period of four (4) weeks under storage conditions at 4°C by measuring the CFU/g of the dried beads. The tests were performed at least in triplicates and one standard deviation was calculated.

The results of Example 12 are shown in Table 20 and all the preservation solutions tested afforded feed additive strain stability during 4 weeks when stored at 4°C.

Table 20

| Preservation solution | Difference CFU/g after 4 weeks (log) |
|---|---|
| S1 | 0 |
| S13 | 0 |
| S14 | 0.1 |
| S15 | 0.1 |

13. Example 13

For each preservation solution the drying and testing was performed at least in triplicates. Beads prepared as in Example 1 were placed in either a preservation solution S1, a preservation solution S16, a preservation solution S17 and a preservation solution S18 with gentle stirring for about 20 minutes. The beads were then placed on a tray dryer in an air dryer at room temperature for about 24 h to obtain semi-dry beads. The semi-dry beads were then placed in a desiccator for about 64 h, in which dry and filtered air was blown. Dry beads having a water activity $a_w$ of $\leq 0.3$ were obtained.

In each case, the strain viability was tested over a period of four (4) weeks under storage conditions at 4°C by measuring the CFU/g of the dried beads. The tests were performed at least in triplicates and one standard deviation was calculated.

The results of Example 13 are shown in Table 21 and all the preservation solutions tested afforded feed additive strain stability during 4 weeks when stored at 4°C.

Table 21

| Preservation solution | Difference CFU/g after 4 weeks (log) |
|---|---|
| S1 | 0 |
| S16 | 0 |
| S17 | 0 |
| S18 | 0.1 |

14. Example 14

For each preservation solution the drying and testing was performed at least in triplicates. Beads prepared as in Example 1 were placed in either a preservation solution S1 and a preservation solution S19 with gentle stirring for about 20 minutes. The beads were then placed on a tray dryer in an air dryer at room temperature for about 24 h to obtain semi-dry beads. The semi-dry beads were then placed in a desiccator for about 64 h, in which dry and filtered air was blown. Dry beads having a water activity $a_w$ of $\leq 0.3$ were obtained.

In each case, the strain viability was tested over a period of four (4) weeks under storage conditions at 4°C by measuring the CFU/g of the dried beads. The tests were performed at least in triplicates and one standard deviation was calculated.

The results are shown in Table 22 and all the preservation solutions tested afforded feed additive strain stability during 4 weeks when stored at 4°C.

Table 22

| Preservation solution | Difference CFU/g after 4 weeks (log) |
| --- | --- |
| S1 | 0.1 |
| S19 | 0.1 |

In brief, the present inventor has surprisingly and unexpectedly observed that a matrix comprising embedded viable *E. coli* as described herein was capable of preserving viability of sufficient bacteria CFU over a given period of time, e.g. 4 weeks, for a commercial use thereof. For example, the matrix was successfully incorporated into a pelleted animal feed such that the animal feed could be stored / transported / handled and eventually administered to an animal while retaining sufficient viable CFU / g of animal feed to provide the beneficial effect normally associated with the bacteria.

It will be understood by those of skill in the art that throughout the present specification, the term "a" used before a term encompasses embodiments containing one or more to what the term refers. It will also be understood by those of skill in the art that throughout the present specification, the term "comprising", which is synonymous with "including," "containing," or "characterized by," is inclusive or open-ended and does not exclude additional, un-recited elements or method steps.

Unless otherwise defined, all technical and scientific terms used herein have the same meaning as commonly understood by one of ordinary skill in the art to which this invention pertains. In the case of conflict, the present document, including definitions will control.

As used in the present disclosure, the terms "around", "about" or "approximately" shall generally mean within the error margin generally accepted in the art. Hence, numerical quantities given herein generally include such error margin such that the terms "around", "about" or "approximately" can be inferred if not expressly stated.

Although the present disclosure has described in considerable detail certain embodiments, variations and refinements are possible and will become apparent to persons skilled in the art in light of the present teachings.

**Claims**

1. A method for providing viable *Escherichia coli* (*E. coli)* embedded in a matrix in form of a particle, the method comprising mixing *E. coli* with alginate, forming particles, contacting the particles with a preservation solution comprising maltodextrin or dextran and further comprising sucrose or trehalose, and drying to obtain a water activity ≤ 0.3.

2. The method of claim 1, wherein said drying includes air drying.

3. The method of claim 2, wherein said drying comprises drying said particles in an air dryer.

4. The method of claim 2, wherein said drying comprises drying said particles in a desiccator.

5. The method of claim 4, wherein said drying comprises a first drying in an air drier and a second drying in a desiccator.

6. The method of claim 1, wherein said drying includes freeze drying.

7. The method of claim 1, wherein said matrix comprises a ratio of the sucrose or trehalose / maltodextrin or dextran of less than 10, wherein the ratio is wt.%/wt.%.

8. The method of claim 7, wherein the ratio is of less than 5.

9. The method of claim 7, wherein the ratio is of about 1.

10. The method of claim 1, wherein said matrix further comprises a salt of L-glutamic acid.

11. The method of any one of claims 1 to 10, wherein the *E. coli* is a non-pathogenic *E. coli.*

**Patentansprüche**

1. Ein Verfahren zur Bereitstellung lebensfähiger *Escherichia coli* (*E. coli*), eingebettet in eine Matrix in Form eines Partikels, wobei das Verfahren Mischen von *E. coli* mit Alginat, Bilden von Partikeln, Inkontaktbringen der Partikel mit einer Konservierungslösung, umfassend Maltodextrin oder Dextran und außerdem umfassend Sucrose oder Trehalose, und Trocknen umfasst, um eine Wasseraktivität ≤ 0,3 zu erhalten.

2. Das Verfahren nach Anspruch 1, wobei besagtes Trocknen Lufttrocknen umfasst.

3. Das Verfahren nach Anspruch 2, wobei besagtes Trocknen ein Trocknen besagter Partikel in einem Lufttrockner umfasst.

4. Das Verfahren nach Anspruch 2, wobei besagtes Trocknen ein Trocknen besagter Partikel in einem Exsikkator umfasst.

5. Das Verfahren nach Anspruch 4, wobei besagtes Trocknen ein erstes Trocknen in einem Lufttrockner und ein zweites Trocknen in einem Exsikkator umfasst.

6. Das Verfahren nach Anspruch 1, wobei besagtes Trocknen Gefriertrocknen umfasst.

7. Das Verfahren nach Anspruch 1, wobei besagte Matrix ein Verhältnis von Sucrose oder Trehalose / Maltodextrin oder Dextran von kleiner als 10 umfasst, wobei das Verhältnis in Gew.-%/Gew.-% ausgedrückt ist.

8. Das Verfahren nach Anspruch 7, wobei das Verhältnis kleiner als 5 ist.

9. Das Verfahren nach Anspruch 7, wobei das Verhältnis etwa 1 beträgt.

10. Das Verfahren nach Anspruch 1, wobei besagte Matrix außerdem ein L-Glutaminsäure-Salz umfasst.

11. Das Verfahren nach einem der Ansprüche 1 bis 10, wobei das *E. coli* Bakterium ein nichtpathogenes *E. coli* Bakterium ist.


**Revendications**

1. Méthode pour obtenir des *Escherichia coli* (*E. coli*) viables incorporées dans une matrice sous la forme d'une particule, la méthode comprenant le mélange d'*E. coli* avec un alginate, la formation de particules, la mise en contact des particules avec une solution de conservation comprenant de la maltodextrine ou du dextrane et comprenant en outre du saccharose ou du tréhalose, et le séchage pour que soit obtenue une activité de l'eau ≤ 0,3.

2. Méthode selon la revendication 1, dans laquelle ledit séchage comprend un séchage à l'air.

3. Méthode selon la revendication 2, dans laquelle ledit séchage comprend le séchage desdites particules dans un séchoir à air.

4. Méthode selon la revendication 2, dans laquelle ledit séchage comprend le séchage desdites particules dans un dessiccateur.

5. Méthode selon la revendication 4, dans laquelle ledit séchage comprend un premier séchage dans un séchoir à air et un deuxième séchage dans un dessiccateur.

6. Méthode selon la revendication 1, dans laquelle ledit séchage comprend une lyophilisation.

7. Méthode selon la revendication 1, dans laquelle ladite matrice comprend un rapport saccharose ou tréhalose / maltodextrine ou dextrane inférieur à 10, lequel rapport est en % en poids / % en poids.

8. Méthode selon la revendication 7, dans laquelle le rapport est inférieur à 5.

**9.** Méthode selon la revendication 7, dans laquelle le rapport est d'environ 1.

**10.** Méthode selon la revendication 1, dans laquelle ladite matrice comprend en outre un sel d'acide L-glutamique.

**11.** Méthode selon l'une quelconque des revendications 1 à 10, dans laquelle les *E. coli* sont des *E. coli* non pathogènes.

**Figure 1**

```
┌─────────────────────────────────────────────┐
│                  20 mins                      │ ╱ 500
│         Beads in preservation solution        │
└─────────────────────────────────────────────┘
```

┌─────────────┐        ↑ ──────────┐   transfer
│  CFU count  │ ◄───────           │
└─────────────┘                    ↓
  ╱
550

```
┌─────────────────────────────────────────────┐
│                 24 hours                      │ ╱ 600
│        Air drier at room temperature          │
│           to obtain semi-dry beads            │
└─────────────────────────────────────────────┘
```

┌──────────────┐       ↑ ──────────┐   transfer
│  aw measure  │ ◄──────           │
└──────────────┘                   ↓
  ╱
650

```
┌─────────────────────────────────────────────┐
│                 64 hours                      │ ╱ 700
│                Desiccator                     │
│            to obtain dry beads                │
└─────────────────────────────────────────────┘
```

800

```
      ↓                          ↓
┌─────────────┐          ┌──────────────┐
│  CFU count  │          │  aw measure  │
└─────────────┘          └──────────────┘
     ╱                         ╱
   750                       760
```

Drying process

**Figure 2**

**Figure 3**

Figure 4

**Figure 5**

**Figure 6**

**Figure 7**

**Figure 8**

**Figure 9**

| Test | Solution code | Replicates | Drying | | | $a_w$ | | Stability per week (CFU/g) | | | | |
|---|---|---|---|---|---|---|---|---|---|---|---|---|
| | | | Before (CFU total) | After (CFU total) | difference (log) | Before | After | 0 | 1 | 2 | 3 | 4 |
| 1 | S1 | A | 3.1E+11 | 1.4E+11 | -0.36 | 0.4502 | 0.1536 | 3.4E+09 | 4.5E+09 | 4.1E+09 | 4.4E+09 | 8.6E+09 |
| | | B | 2.1E+11 | 1.4E+11 | -0.17 | 0.4831 | 0.1740 | 3.8E+09 | 5.4E+09 | 4.8E+09 | 3.2E+09 | 4.8E+09 |
| | | C | 3.9E+11 | 1.4E+11 | -0.43 | 0.4871 | 0.1671 | 3.8E+09 | 5.0E+09 | 4.3E+09 | 4.9E+09 | 5.5E+09 |
| | S2 | A | 2.3E+11 | 2.8E+09 | -1.92 | 0.2549 | 0.0616 | 1.9E+08 | 9.9E+07 | 1.3E+08 | 1.9E+08 | 3.5E+08 |
| | | B | 1.8E+11 | 8.1E+09 | -1.34 | 0.2836 | 0.0561 | 5.4E+08 | 6.2E+08 | 4.5E+08 | 8.2E+08 | 5.4E+08 |
| | | C | 2.9E+11 | 5.3E+09 | -1.74 | 0.2961 | 0.0434 | 3.4E+08 | 1.1E+08 | 1.5E+08 | 2.0E+08 | 4.6E+08 |
| | S3 | A | 2.3E+11 | 1.0E+11 | -0.34 | 0.4215 | 0.1274 | 4.3E+09 | 1.8E+09 | 1.5E+09 | 4.5E+09 | 4.4E+09 |
| | | B | 2.3E+11 | 2.5E+10 | -0.96 | 0.4011 | 0.1437 | 1.1E+09 | 1.2E+09 | 1.7E+09 | 1.5E+09 | 2.3E+09 |
| | | C | 3.1E+11 | 9.1E+10 | -0.53 | 0.4459 | 0.1791 | 3.9E+09 | 5.9E+09 | 5.0E+09 | 3.2E+09 | 6.1E+09 |
| | S4 | A | 2.9E+11 | 2.4E+11 | -0.08 | 0.4687 | 0.1376 | 6.6E+09 | 6.3E+09 | 4.9E+09 | 5.0E+09 | 6.3E+09 |
| | | B | 2.6E+11 | 1.6E+11 | -0.20 | 0.4848 | 0.1429 | 4.6E+09 | 4.0E+09 | 5.3E+09 | 5.6E+09 | 4.2E+09 |
| | | C | 3.9E+11 | 3.5E+11 | -0.04 | 0.5114 | 0.1463 | 9.7E+09 | 5.8E+09 | 6.6E+09 | 6.2E+09 | 6.2E+09 |
| 2 | S1 | A | 2.5E+11 | 1.5E+11 | -0.24 | 0.5219 | 0.2174 | 3.7E+09 | 7.4E+09 | 5.8E+09 | 6.6E+09 | 5.9E+09 |
| | | B | 4.3E+11 | 2.0E+11 | -0.34 | 0.5582 | 0.2299 | 5.0E+09 | 5.0E+09 | 4.8E+09 | 6.9E+09 | 7.7E+09 |
| | | C | 3.0E+11 | 1.9E+11 | -0.20 | 0.5255 | 0.2416 | 4.9E+09 | 5.1E+09 | 6.3E+09 | 4.9E+09 | 5.9E+09 |
| | S5 | A | 3.6E+11 | 2.0E+11 | -0.24 | 0.5016 | 0.2470 | 5.4E+09 | 1.0E+10 | 8.3E+09 | 4.7E+09 | 6.4E+09 |
| | | B | 4.3E+11 | 2.6E+11 | -0.22 | 0.5011 | 0.2413 | 6.5E+09 | 5.8E+09 | 6.5E+09 | 2.8E+09 | 8.0E+09 |
| | | C | 2.8E+11 | 3.2E+11 | 0.07 | 0.5864 | 0.2592 | 8.1E+09 | 7.4E+09 | 5.6E+09 | 5.7E+09 | 9.4E+09 |
| | S6 | A | 2.8E+11 | 4.8E+11 | 0.23 | 0.7551 | 0.2747 | 1.2E+10 | 9.4E+09 | 6.2E+09 | 8.1E+09 | 8.8E+09 |
| | | B | 3.8E+11 | 2.5E+11 | -0.19 | 0.5017 | 0.2539 | 6.2E+09 | 5.6E+09 | 7.1E+09 | 5.9E+09 | 7.5E+09 |
| | | C | 2.8E+11 | 1.3E+11 | -0.33 | 0.5065 | 0.2511 | 3.3E+09 | 4.6E+09 | 7.2E+09 | 4.7E+09 | 5.0E+09 |
| | S7 | A | 3.3E+11 | 2.8E+11 | -0.07 | 0.5929 | 0.2840 | 7.0E+09 | 1.0E+10 | 8.5E+09 | 6.9E+09 | 1.0E+10 |
| | | B | 3.8E+11 | 2.6E+11 | -0.16 | 0.5190 | 0.3027 | 6.5E+09 | 8.1E+09 | 4.8E+09 | 4.7E+09 | 8.6E+09 |
| | | C | 3.1E+11 | 2.7E+11 | -0.06 | 0.5104 | 0.3086 | 6.6E+09 | 4.9E+09 | 4.9E+09 | 4.4E+09 | 6.2E+09 |
| 3 | S1 | A | 2.0E+11 | 1.6E+11 | -0.10 | 0.4418 | 0.2399 | 4.5E+09 | 5.6E+09 | 2.9E+09 | 4.1E+09 | 3.2E+09 |
| | | B | 2.5E+11 | 1.7E+11 | -0.17 | 0.4611 | 0.2366 | 4.9E+09 | 5.4E+09 | 4.0E+09 | 5.3E+09 | 4.6E+09 |
| | | C | 2.1E+11 | 1.8E+11 | -0.07 | 0.4561 | 0.2468 | 5.1E+09 | 5.3E+09 | 3.1E+09 | 4.9E+09 | 4.8E+09 |
| | S0 | A | 1.8E+11 | 2.9E+05 | -5.79 | 0.3066 | 0.0391 | 3.8E+04 | 4.4E+05 | 3.6E+04 | 4.7E+05 | 4.7E+05 |
| | | B | 2.1E+11 | 1.0E+06 | -5.31 | 0.3343 | 0.0362 | 1.4E+05 | 7.1E+05 | 9.4E+05 | 2.8E+06 | 1.5E+08 |
| | | C | 1.7E+11 | 3.2E+06 | -4.73 | 0.3510 | 0.0356 | 4.2E+05 | 8.5E+05 | 7.2E+05 | 9.4E+05 | 3.1E+06 |
| | S8 | A | 3.2E+11 | 4.5E+10 | -0.85 | 0.3631 | 0.0680 | 1.5E+09 | 1.9E+09 | 3.4E+09 | 3.2E+09 | 2.5E+09 |
| | | B | 2.3E+11 | 4.7E+10 | -0.69 | 0.3585 | 0.0565 | 1.5E+09 | 1.1E+09 | 1.6E+09 | 2.2E+09 | 1.9E+09 |
| | | C | 3.0E+11 | 8.6E+10 | -0.54 | 0.3777 | 0.0614 | 2.8E+09 | 1.9E+09 | 3.0E+09 | 4.2E+09 | 3.3E+09 |
| | S9 | A | 2.3E+11 | 1.5E+11 | -0.17 | 0.4625 | 0.2392 | 4.5E+09 | 4.5E+09 | 3.6E+09 | 3.8E+09 | 4.0E+09 |
| | | B | 3.0E+11 | 1.7E+11 | -0.25 | 0.4433 | 0.2862 | 4.8E+09 | 3.9E+09 | 3.8E+09 | 4.4E+09 | 4.5E+09 |
| | | C | 1.9E+11 | 1.4E+11 | -0.14 | 0.4472 | 0.3011 | 4.0E+09 | 5.0E+09 | 4.3E+09 | 4.8E+09 | 2.9E+09 |
| 4 | S1 | A | 3.1E+11 | 2.3E+11 | -0.14 | 0.4589 | 0.1150 | 5.7E+09 | 5.8E+09 | 4.5E+09 | 5.7E+09 | 4.9E+09 |
| | | B | 3.9E+11 | 3.0E+11 | -0.11 | 0.4655 | 0.1267 | 7.6E+09 | 6.0E+09 | 4.5E+09 | 6.6E+09 | 4.5E+09 |
| | | C | 4.1E+11 | 3.1E+11 | -0.12 | 0.5011 | 0.1272 | 7.8E+09 | 6.3E+09 | 4.7E+09 | 4.6E+09 | 6.1E+09 |
| | S10 | A | 2.9E+11 | 3.2E+11 | 0.04 | 0.4617 | 0.1279 | 8.3E+09 | 6.6E+09 | 5.6E+09 | 5.9E+09 | 6.5E+09 |
| | | B | 4.9E+11 | 3.1E+11 | -0.20 | 0.4977 | 0.1363 | 7.6E+09 | 7.5E+09 | 6.9E+09 | 5.9E+09 | 6.2E+09 |
| | | C | 4.2E+11 | 3.7E+11 | -0.06 | 0.5117 | 0.1417 | 9.0E+09 | 7.8E+09 | 8.5E+09 | 6.6E+09 | 6.3E+09 |
| | S11 | A | 2.8E+11 | 1.8E+11 | -0.20 | 0.4289 | 0.1567 | 6.2E+09 | 3.9E+09 | 4.1E+09 | 5.5E+09 | 3.0E+09 |
| | | B | 3.3E+11 | 1.8E+11 | -0.27 | 0.4003 | 0.2199 | 6.2E+09 | 5.0E+09 | 4.0E+09 | 3.1E+09 | 2.6E+09 |
| | | C | 3.6E+11 | 2.1E+11 | -0.24 | 0.4287 | 0.2259 | 7.6E+09 | 8.1E+09 | 5.0E+09 | 6.3E+09 | 2.4E+09 |
| | S12 | A | 3.8E+11 | 6.1E+11 | 0.20 | 0.5165 | 0.1639 | 1.6E+10 | 1.2E+10 | 1.1E+10 | 1.1E+10 | 9.0E+09 |
| | | B | 4.4E+11 | 5.5E+11 | 0.10 | 0.4994 | 0.1719 | 1.5E+10 | 1.2E+10 | 1.1E+10 | 1.3E+10 | 9.7E+09 |
| | | C | 3.9E+11 | 4.3E+11 | 0.04 | 0.4652 | 0.1596 | 1.1E+10 | 8.6E+09 | 1.1E+10 | 7.9E+09 | 5.2E+09 |
| 5 | S1 | A | 4.5E+11 | 2.4E+11 | -0.28 | 0.4816 | 0.1677 | 5.6E+09 | 4.5E+09 | 3.1E+09 | 5.5E+09 | 5.0E+09 |
| | | B | 3.7E+11 | 3.1E+11 | -0.09 | 0.5490 | 0.1824 | 7.3E+09 | 6.8E+09 | 7.0E+09 | 7.0E+09 | 7.6E+09 |
| | | C | 4.0E+11 | 2.7E+11 | -0.18 | 0.4739 | 0.1821 | 6.1E+09 | 5.9E+09 | 6.4E+09 | 5.5E+09 | 5.9E+09 |
| | S13 | A | 3.7E+11 | 2.5E+11 | -0.17 | 0.6726 | 0.2386 | 6.1E+09 | 5.6E+09 | 6.2E+09 | 6.0E+09 | 6.7E+09 |
| | | B | 3.4E+11 | 2.5E+11 | -0.14 | 0.4740 | 0.2422 | 6.1E+09 | 7.4E+09 | 5.0E+09 | 6.8E+09 | 5.9E+09 |
| | | C | 4.2E+11 | 3.0E+11 | -0.15 | 0.5405 | 0.2602 | 7.3E+09 | 6.3E+09 | 3.7E+09 | 7.5E+09 | 6.3E+09 |
| | S14 | A | 3.1E+11 | 2.0E+11 | -0.19 | 0.4778 | 0.1371 | 6.5E+09 | 4.9E+09 | 3.5E+09 | 5.3E+09 | 4.7E+09 |
| | | B | 4.2E+11 | 2.6E+11 | -0.20 | 0.4295 | 0.1184 | 8.4E+09 | 8.6E+09 | 6.2E+09 | 6.6E+09 | 5.0E+09 |
| | | C | 4.2E+11 | 2.0E+11 | -0.33 | 0.4864 | 0.1434 | 6.3E+09 | 8.4E+09 | 8.2E+09 | 9.0E+09 | 6.5E+09 |
| | S15 | A | 4.2E+11 | 3.5E+11 | -0.08 | 0.5324 | 0.1938 | 9.5E+09 | 8.5E+09 | 8.3E+09 | 1.1E+10 | 8.3E+09 |
| | | B | 4.5E+11 | 3.4E+11 | -0.12 | 0.4612 | 0.1854 | 9.5E+09 | 9.1E+09 | 8.3E+09 | 7.9E+09 | 7.6E+09 |
| | | C | 4.5E+11 | 4.6E+11 | 0.01 | 0.5118 | 0.2164 | 1.2E+10 | 9.1E+09 | 8.9E+09 | 8.4E+09 | 7.3E+09 |
| 6 | S1 | A | 2.9E+11 | 2.9E+11 | 0.00 | 0.7314 | 0.1529 | 7.1E+09 | 6.5E+09 | 7.3E+09 | 6.7E+09 | 6.7E+09 |
| | | B | 3.5E+11 | 3.7E+11 | 0.02 | 0.8990 | 0.1590 | 9.0E+09 | 8.3E+09 | 8.9E+09 | 9.4E+09 | 8.2E+09 |
| | | C | 3.5E+11 | 2.8E+11 | -0.11 | 0.5714 | 0.1540 | 6.9E+09 | 5.3E+09 | 5.5E+09 | 6.8E+09 | 6.2E+09 |
| | S16 | A | 3.5E+11 | 3.2E+11 | -0.04 | 0.5313 | 0.1219 | 8.1E+09 | 8.9E+09 | 1.3E+10 | 1.2E+10 | 9.0E+09 |
| | | B | 4.1E+11 | 4.9E+11 | 0.08 | 0.5198 | 0.1326 | 1.2E+10 | 1.1E+10 | 1.3E+10 | 1.7E+10 | 1.2E+10 |
| | | C | 3.3E+11 | 4.9E+11 | 0.18 | 0.6745 | 0.1524 | 1.3E+10 | 1.1E+10 | 9.0E+09 | 1.3E+10 | 1.1E+10 |
| | S17 | A | 3.0E+11 | 1.9E+11 | -0.21 | 0.5994 | 0.0372 | 5.3E+09 | 6.5E+09 | 6.8E+09 | 5.9E+09 | 7.3E+09 |
| | | B | 3.8E+11 | 2.9E+11 | -0.12 | 0.9267 | 0.0439 | 8.2E+09 | 8.5E+09 | 9.8E+09 | 8.9E+09 | 1.1E+10 |
| | | C | 3.0E+11 | 2.2E+11 | -0.13 | 0.7010 | 0.0371 | 6.5E+09 | 7.0E+09 | 5.9E+09 | 5.8E+09 | 5.8E+09 |
| | S18 | A | 2.5E+11 | 3.7E+11 | 0.17 | 0.5388 | 0.1469 | 9.6E+09 | 7.9E+09 | 1.2E+10 | 7.8E+09 | 6.3E+09 |
| | | B | 3.2E+11 | 4.4E+11 | 0.14 | 0.5327 | 0.1771 | 1.1E+10 | 1.3E+10 | 8.7E+09 | 1.1E+10 | 1.1E+10 |
| | | C | 2.5E+11 | 4.6E+11 | 0.26 | 0.5359 | 0.2048 | 1.2E+10 | 1.1E+10 | 9.4E+09 | 1.1E+10 | 9.4E+09 |
| 7 | S1 | A | 3.5E+11 | 3.1E+11 | -0.05 | 0.6498 | 0.1473 | 8.1E+09 | 8.0E+09 | - | 6.9E+09 | 5.9E+09 |
| | | B | 3.5E+11 | 3.7E+11 | 0.03 | 0.4659 | 0.1387 | 9.7E+09 | 8.8E+09 | - | 7.3E+09 | - |
| | | C | 3.5E+11 | 2.9E+11 | -0.08 | 0.8656 | 0.1871 | 7.6E+09 | 9.9E+09 | - | 9.2E+09 | 7.9E+09 |
| | S19 | A | 3.0E+11 | 2.5E+11 | -0.08 | 0.6582 | 0.1401 | 6.8E+09 | 5.7E+09 | - | 4.3E+09 | 5.7E+09 |
| | | B | 3.5E+11 | 2.6E+11 | -0.13 | 0.5270 | 0.1194 | 7.0E+09 | 7.2E+09 | - | 5.8E+09 | 6.0E+09 |
| | | C | 3.4E+11 | 2.2E+11 | -0.19 | 0.4994 | 0.1280 | 6.0E+09 | 5.0E+09 | - | 4.6E+09 | 4.5E+09 |

Figure 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8097245 B
- US 20130296165 A

**Non-patent literature cited in the description**

- **HONG et al.** *FEMS Microbiology Reviews,* 2005, vol. 29, 813-835
- **RHODES.** Exploitation of microorganisms. Chapman & Hall, 1993, 411-439
- **FONT DE VALDEZ et al.** *Cryobiology,* 1983, vol. 20, 560-566
- **LIEVENSE et al.** *dv Biochem Eng Biotechnol.,* 1994, vol. 51, 71-89
- **ISLAM et al.** *J. Microbiol. Biotechnol.,* 2010, vol. 20, 1367-1377
- **EFIUVWEVWERE et al.** *Appl. Microbiol. Biotechnol.,* 1999, vol. 51, 100-104
- **WYNANT et al.** *Biocatalysis and Biotransformation,* 2009, vol. 27 (5-6), 348-359
- **YOUG et al.** *Biotechnol Bioeng.,* 05 September 2006, vol. 95 (1), 76-83
- **SON ; TAYLOR.** *Curr. Protoc. Microbiol.,* 2012, vol. 27, 5A.4.1-5A.4.9